# FASCICULE DE BREVET EUROPEEN

(11) **EP 0 156 712 B1**
(45) Date de publication et mention de la délivrance du brevet: **14.08.1996**
(21) Numéro de dépôt: 85400444.7
(22) Date de dépôt: 07.03.1985
(51) Int. Cl.: A61K 39/29, C12N 15/36, C12N 15/85, C12N 5/00

(54) **Composition utile pour la fabrication de vaccins contenant des particules portant l'antiqène de surface du virus de l'hépatite B et le récepteur de l'albumine sérique humaine polymérisée, cellules animales capables de produire de telles particules et procédé pour leur obtention**
Zubereitung für die Herstellung von Hepatitis B Virus-Oberflächenantigen und polymerisierten Humanserumalbuminrezeptor tragende Partikel enthaltenden Vakzinen, tierische Zellen für die Herstellung von diesen Partikeln und Verfahren für ihre Erhaltung
Useful composition for the manufacture of vaccines containing particles bearing a hepatitis B virus surface antigen and polymerized human seric albumin receptor, animal cells able toproduce such particles and process for their obtention

(30) Priorité: 07.03.1984 FR 8403564
(43) Date de publication de la demande: 02.10.1985
(60) Demande divisionnaire: 95120494.0
(73) Titulaire: INSTITUT PASTEUR, F-75724 Paris Cédex 15 (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75700 Paris Cedex 07 (FR)
(72) Inventeur: Sobczak, Eliane, F-75011 Paris (FR); Malpiece, Yves, F-80000 Amiens (FR); Michel, Marie-Louise, F-75018 Paris (FR); Tiollais, Pierre, F-75013 Paris (FR); Streeck, Rolf E., F-75016 Paris (FR)
(74) Mandataire: Gutmann, Ernest

(56) Documents cités:
- EP-A- 0 038 765
- PROCEEDINGS OF THE NATL. ACADEMY OF SCIENCES USA, vol. 81, décembre 1984, Washington, DC (US); M.L. MICHEL et al., pp. 7708-7712
- BIOLOGICAL ABSTRACTS, vol. 77, no. 2, 1984, Philadelphia, PA (US); A.S. MACHIDA et al., AN 12366
- BIOLOGICAL ABSTRACTS, vol. 68, no. 1, 1979, Philadephia, PA (US); I. MITSUNOBU et al., AN 3690
- CHEMICAL ABSTRACTS, vol. 95, no. 13, 28 septembre 1981, Columbus, OH (US); D.R. MILICH et al., p. 500, AN 113155j
- CHEMICAL ABSTRACTS, vol. 100, no. 21, 21 mai 1984, Columbus, OH (US); A. ALBERTI et al., p. 468, AN 172783g
- MOLECULAR & CELLULAR BIOLOGY, vol. 1, 1981; pp. 854-864
- THE EMBO JOURNAL, vol. 1, no. 12, 1982; pp. 1573-1578
- THE EMBO JOURNAL, vol. 2, no. 5, 1983; pp. 669-673

## Description

L'invention concerne une composition utile pour la fabrication de vaccins, contenant ou formée par des particules polypeptidiques sensiblement sphériques, au moins en ce qui concerne la pupart d'entre elles, ces particules ayant les propriétés immunogéniques et immunologiques caractéristiques de l'antigène de surface du virus de l'hépatite virale B. Cet antigène est souvent désigné par l'abréviation HBsAg ou encore plus simplement HBs. Elle concerne également des lignées cellulaires eucaryotes, de préférence animales, capables d'excréter dans leur milieu de culture des particules polypeptidiques du genre sus-indiqué avec des rendements de production élevés et des moyens, notamment des vecteurs modifiés, permettant d'obtenir de telles lignées cellulaires.

On rappellera tout d'abord que le sérum des porteurs chroniques du virus de l'hépatite B (HBV) contient des enveloppes virales vides sous forme de particules ou filaments de 22 nm de diamètre et parfois des virions complets infectieux, particules sphériques de 42 nm. L'enveloppe virale porte l'antigène de surface (HBsAg) et la présence de virions infectieux s'accompagne généralement d'un antigène soluble appelé antigène (HBeAg).

La composition polypeptidique de l'enveloppe virale a été très étudiée (Robinson W.S. (1977) Ann. Rev. Microbiol. 31, 357-377). Elle comprend un polypeptide majeur présent sous forme glycosylée (GP29) et non glycosylée (P25) et au moins trois polypeptides mineurs appelés GP33, GP36 et P41. Les polypeptides GP33 et GP36 auraient la même séquence en acides aminés. GP36 posséderait seulement un résidu glucidique supplémentaire (Stibbe W. et Gerlich W.H. (1983) J. Virology 46, 626-628). La quantité relative des protéines mineures par rapport au polypeptide majeur varie d'un plasma à l'autre. Elle est très supérieure dans les sérums HBeAg positifs riches en particules virales. Les polypeptides GP33 et GP36 peuvent alors représenter plusieurs % des protéines de l'enveloppe alors qu'ils représentent moins de 1 % dans les sérums non infectieux HBeAg négatifs (Stibbe W. et Gerlich W.H. (1982) Virology 123, 436-442).

Le polypeptide majeur de l'enveloppe est constitué de 226 acides aminés et est codé par le gène S. La séquence polypeptidique de GP33 et GP36 serait codée par le gène S et une partie de la région pré-S. Dans cette hypotnèse, cette séquence poylpeptidique aurait la même extrémité C-terminale que le polypeptide majeur et contiendrait une séquence supplémentaire de 55 acides aminés en position N-terminale (Stibbe W. et Gerlich W.H. (1983), J. Virology 46, 626-628).

Le brevet européen 0 020 251 décrit un produit formant une bande sur un gel d'électrophorèse, cette bande correspondant vraisemblablement à une protéine ayant un poids moléculaire de l'ordre de 27000, dépourvue de séquence "leader".

Récemment, il a été montré que les particules virales isolées d'un sérum HBeAg positif contiennent un récepteur pour l'albumine humaine polymérisée (pHSA) (Machica A. et al (1983) Gastroenterology 85, 268-274). Ce récepteur serait porté par les polypeptides GP33 et GP36. Grâce à ce récepteur, la pHSA formerait un pont entre la particule virale et l'hépatocyte, permettant ainsi l'attachement du virus et sa pénétration dans la cellule hépatique. L'apparition des anticorps anit-récepteurs serait essentielle dans le processus d'élimination ("clearance") du virus. En effet, la séro-conversion HBeAg/anti-HBe, étape initiale de la guérison, s'accompagne de l'apparition de ces anticorps, alors que ceux-ci sont absents au cours de l'évolution vers la chronicité (Pontisso P. et al (1983) J. of Virological Methods 6, 151-159).

Parallèlement, on observe la disparition des antigènes correspondants au récepteur de la pHSA, ce qui paraît témoigner de ce que l'expression du gène correspondant n'intervient normalement que dans des situations biologiques précises (notamment au cours de l'étape de réplication du virus). C'est ce que corrobore encore l'analyse des enveloppes vides naturelles du virus de l'hépatite B, telles qu'elles sont contenues dans des préparations de vaccins actuellement commercialisées et obtenues à partir du sérum sanguin de donneurs qui, dans le passé, ont été exposés au virus de l'hépatite B. En effet, l'analyse par électrophorèse en gel de polyacrylamide en présence de dodécylsulfate de sodium (SDS) des polypeptides obtenus après dissociation des particules naturelles à 100°C pendant 5 minutes, en présence de dithiothréitol (DTT), ne révèle pas la présence de polypeptides ayant des poids moléculaires élevés, notamment de l'ordre de 34.000. De même, la présence de tels polypeptides à poids moléculaires élevés n'a pas été observée dans les compositions de particules possédant les propriétés immunologiques et immunogéniques de l'antigène HBs, telles qu'elles ont été obtenues par transformation de cellules animales transformées par les techniques du génie génétiques au moyen des vecteurs qui ont été décrits par exemple dans la demande de brevet européen n° 38.765.

L'invention a pour but de fournir des compositions de vaccins ayant des propriétés de protection renforcées à l'égard du virus de l'hépatite B. Elle a encore pour but de fournir des lignées cellulaires transformées par les techniques du génie génétique, qui peuvent être maintenues en culture et qui sont aptes à excréter les principes actifs de ces compositions de vaccins renforcées dans leur milieu de culture, et ce avec des rendements de production beaucoup plus élevés que ceux que permettent la plupart des cultures génétiquement transformées dont on dispose à ce jour. Enfin elle a pour but de fournir des moyens (vecteurs et procédé) permettant l'obtention de telles lignées cellulaires à partir de lignées de cellules eucaryotes, notamment de mammifères, du genre de celles qui sont capables d'être maintenues en culture.

L'invention résulte de la constatation qui a été faite qu'il était possible d'induire l'expression des séquences génétiques qui, dans le génome du virus de l'hépatite B codent pour le récepteur de la pHSA, dans des conditions pourtant très éloignées de celles qui prévalent dans les situations biologiques précises qui ont été évoquées plus haut. Enfin, il a été constaté que l'association dans la même composition de vaccin de l'antigène HBs avec des fragments polypeptidiques portant un déterminant antigènique paraissant correspondre à ce récepteur, conduisant aux compositions de vaccin renforcées dont il a été question plus haut.

La composition utile pour la fabrication de vaccins selon l'invention qui contient des particules polypeptidiques sensiblement sphériques (ou qui est formée par ces particules), au moins en ce qui concerne la plupart d'entre elles (sinon toutes), qui ont les propriétés immunogéniques et immunologiques caractéristiques de l'antigène HBsAg, qui ont des tailles de 18 à 25 nm, notamment de 20 à 22 nm, et des densités permettant leur isolement dans une zone de densité de 1,20-1,22 g/ml dans un gradient de densité à base de CsCl, et un niveau de pureté totale pour ce qui est de l'absence de toute particule de Dane et d'antigène HBe, y inclus HBc, est plus particulièrement caractérisée par le fait que lesdites particules sphériques contiennent également un récepteur pour l'albumine humaine polymérisée, ces particules étant susceptibles d'être obtenues parmi les produits d'excrétion de cellules CHO transformées par un vecteur contenant les régions S et pré-S du génome du virus de l'hépatite B sous le contrôle d'un promoteur exogène efficace. Les particules polypeptidiques de l'invention contiennent en outre des polypeptides ayant des poids moléculaires de l'ordre de 34.000 daltons, en une proportion supérieure à 10%, et de préférence supérieure à 20% de la quantité totale du polypeptide constitutif des susdites particules. De préférence encore, la composition selon l'invention est exempte de tout composant d'origine humaine, tel qu'on en trouve dans les sérums sanguins humains. Il s'agit essentiellement de compositions de particules ayant les propriétés immunogéniques de l'antigène HBsAg et du récepteur de la pHSA qui sont excrétés dans leur milieu de culture par des lignées cellulaires maintenues en culture et qui ont au préalable été transformées par un vecteur contenant les séquences codantes appropriées, la transformation ayant été réalisée dans des conditions permettant l'expression effective des séquences peptidiques portant les sites immunogènes aussi bien de l'antigène HBs que du récepteur de la pHSA.

Des vecteurs appropriés à la transformation des lignées cellulaires CHO en culture pour rendre celles-ci aptes à produire les particules immunogéniques sus-indiquées, contiennent une séquence d'ADN codant pour les régions S et pré-S du génome du virus de l'hépatite virale B, ladite séquence d'ADN étant, à l'intérieur de ce vecteur, placée sous le contrôle direct d'un promoteur exogène dont est connue la capacité de permettre l'initiation efficace de la transcription des gènes directement sous son contrôle dans les cellules CHO, auxquelles lesdits vecteurs sont destinés. On se reportera par exemple à l'article de Galibert et Col., 1979, Nature, vol. 281, p. 646-650, pour ce qui est de ladite séquence d'ADN. Elle comprend notamment le gène S proprement dit précédé de la séquence pré-S comportant approximativement 165 triplets et dont les premiers nucléotides apparaissent dans la formulation partielle desdites séquences indiquée ci-dessous :

preS          S
Met           Met
ATCTACAGCATGGG--ACATGGAG--
2848          155

Le promoteur exogène mis en oeuvre est distinct ou étranger vis-à-vis du promoteur "endogène", normalement associé aux gènes S et pré-S dans le génome du virus de l'hépatite B. Lorsque ces cellules sont originaires du singe, il est avantageux d'avoir recours à un promoteur issu du virus SV40, dont est connue la capacité de permettre l'initiation efficace de la transcription de gènes adjacents dans des cellules de singe. Avantageusement, ce promoteur correspond au promoteur "précoce" du virus SV40, lequel contrôle normalement l'expression de l'antigène "petit T" ("small T antigen") et également de l'antigène "grand T" ("large T antigen").

L'invention ne se limite cependant pas à l'utilisation de ce promoteur particulier, bien que celui-ci ait donné des résultats particulièrement favorables eu égard à la production par les cellules transformées de polypeptides portant les déterminants immunogéniques caractéristiques de l'antigène HBs et d'un récepteur de la pHSA, et à leur excrétion dans le milieu de culture utilisé. On peut également avoir recours par exemple au promoteur tardif de SV40 (qui contrôle l'expression des protéines VP1, VP2 et VP3). On peut se reporter à la carte de restriction du virus SV40 (J. Tooze, Ed. DNA Tumor Viruses, Cold Spring Harbor Laboratory, Cold Spring Harbor, N.Y., 1980, chaps. 2-5), pour apprécier les positions relatives de ces promoteurs et des gènes codant pour les différents antigènes qui leur sont associés.

Il va de soi que l'on peut substituer aux promoteurs de SV40 tout autre type de promoteur connu comme possédant ou dont pourrait être découverte la capacité de promouvoir la transcription dans les lignées cellulaires mises en oeuvre desdites séquences codant pour les susdites régions S et pré-S, dès lors qu'elles seraient placées sous leur contrôle, avec pour résultat l'incorporation de ces séquences avec ce promoteur dans le génome des cellules réceptrices et/ou la capacité conférée aux cellules réceptrices ainsi transformées de synthétiser et d'excréter des quantités substantielles de polypeptides ayant les propriétés immunogéniques de HBsAg et du récepteur de la pHSA, la capacité ainsi acquise étant ensuite transmissible aux générations successives issues de ces cellules.

Lesdites lignées transformées seront dites "stables" lorsque le caractère acquis par les lignées cellulaires selon l'invention de synthétiser les susdits polypeptides se transmet d'une génération de cellules à l'autres, sur au moins 10 générations.

A titre d'autres promoteurs susceptibles d'être utilisés, on mentionnera par exemple le promoteur précoce du polyome ou des promoteurs LTR de différents rétrovirus ou encore le promoteur EA de l'adénovirus.

Comme il est bien connu, les promoteurs prélevés sur les génomes des virus dont ils sont originaires sont de préférence accompagnés des "séquences" activatrices qui normalement les précèdent (par rapport au sens de la transcription des séquences géniques normalement placées sous leur contrôle). A titre d'exemple de séquences activatrices, on peut se référer à l'article de Science, 1983, vol. 219, pages 626 à 631, et Nature, 1982, vol. 295, pages 568 à 572.

Avantageusement, la susdite séquence d'ADN codant pour les susdites régions pré-S et S est placée, immédiatement derrière un fragment d'ADN constitué par le promoteur et la séquence activatrice permettant la transcription normale de la séquence pré-S ou S. Le susdit fragment comprend notamment de 300 à 400 paires de bases selon le type de promoteur et de séquences activatrices utilisées.

De préférence encore, le vecteur selon l'invention contient en outre une séquence d'ADN ou un marqueur, tel qu'une enzyme, ce marqueur étant lui-même sous le contrôle d'un promoteur distinct plus faible que le premier promoteur mentionné ci-dessus. Ce marqueur est de préférence constitué par un gène ou séquence d'ADN codant pour la dihydrofolate réductase (dhfr). Il est à noter que ce marqueur se prête avec un avantage particulier à l'amplification du nombre de copies de ce vecteur dans les cellules qu'il est capable de transformer, dans les conditions qui seront indiquées plus loin.

Le second promoteur est dans la plupart des cas également un promoteur exogène. Il peut cependant également être constitué par l'un des promoteurs naturels contenus dans le génome de l'hépatite virale B. Un promoteur particulièrement préféré est celui qui provient de la LTR (séquence appelée "Long Terminal Repeat" décrite dans Nature, 1981, 294, 228-232) du virus de la tumeur mammaire de la souris (MMTV).

L'invention concerne encore les lignées cellulaires transformées par des vecteurs tels qu'ils ont été définis ci-dessus et qui sont aptes à excréter dans leur milieu de culture les particules immunogènes également définies ci-dessus. En particulier, elle concerne des lignées cellulaires ayant une capacité de production d'au moins 1 microgramme, et de préférence d'au moins 10 microgrammes d'antigène HBsAg par 10⁶ cellules et par 24 heures. L'invention concerne en particulier des lignées caractérisées par des ARN-HBV-spécifiques qui ont des tailles supérieures à 2,1 kb, notamment de l'ordre de 2,2 à 2,8 kb, de préférence 2,6 kb, que l'on peut y détecter lorsque s'effectue la transcription des régions S et pré-S, normalement initiée dans la région pré-S. Des lignées préférées sont celles dans lesquelles on peut également détecter des ARN-HBV-spécifiques ayant des tailles supérieures à 2,1 kb, notamment de l'ordre de 2,2 à 2,8 kb, et de préférence encore de l'ordre de 2,4-2,6 kb. En général, on détectera simultanément des ARN-HBV-spécifique ayant des tailles de l'ordre de 2,1 kb, tel que celui qui résulte de la transcription des régions S et pré-S, normalement initiés dans la région pré-S sous le contrôle de promoteur endogène, dans les conditions biologiques particulières auxquelles il était fait référence plus haut (notamment au cours de la réplication du virus de l'hépatite B) et des ARN-HBV-spécifiques ayant des tailles correspondant aux valeurs plus élevées qui ont été indiquées.

De préférence, les lignées selon l'invention sont formées de cellules de mammifères, notamment de cellules CHO.

L'invention concerne encore un procédé de production de telles lignées cellulaires susceptibles d'être maintenus en culture, ce procédé comportant la transformation de ces lignées avec un vecteur tel que défini ci-dessus et l'isolement de celles des cultures qui expriment simultanément les séquences codant pour les régions S et pré-S du génome du virus de l'hépatite B.

De préférence, on a recours au vecteur complété qui comprend le marqueur sous le contrôle d'un second promoteur, la culture des lignées transformées étant alors réalisée en présence d'un inhibiteur du marqueur, la teneur de cet inhibiteur dans le milieu de culture étant réglée à une concentration suffisante pour provoquer une amplification du nombre de copies du gène codant pour le marqueur dans certaines au moins des colonies mises en culture, amplification qui permet à la fois la sélection de colonies résistantes et l'obtention de clones cellulaires secréteurs de quantités accrues des particules présentant des activités immunogènes caractéristiques de HBsAg et d'un récepteur de la pHSA.

Avantageusement, le marqueur est constitué par une séquence d'ADN (ou gène) codant pour la dhfr et l'inhibiteur est constitué par le méthotrexate.

L'utilisation d'un second promoteur plus faible que le premier favorise l'amplification du nombre des copies de la séquence d'ADN codant pour HBsAg et le récepteur de la pHSA. En effet ce sont en général les clones qui contiennent le plus grand nombre de copies de la séquence codant pour le marqueur et, par conséquent, dans les lignées cellulaires préférées de l'invention le plus grand nombre des séquences codant pour les régions pré-S et S, qui sont à même de survivre dans le milieu de culture contenant l'inhibiteur.

Les clones efficaces sont avantageusement sélectionnés dans les milieux de culture contenant de 0,5 à 40 microgrammes de méthotrexate, notamment de 1 à 25 microgrammes. Il est souhaitable de réaliser une pré-amplification dans des milieux plus pauvres en inhibiteur, notamment de 5 à 150 nM en méthotrexate, les clones sélectionnés au terme de cette première étape étant ensuite mis en culture dans des milieux contenant les teneurs plus élevées sus-indiquées en méthotrexate.

Des caractéristiques complémentaires de l'invention apparaîtront encore au cours de la description qui suit de la construction préférée des vecteurs mis en oeuvre pour la transformation de lignées cellulaires d'origine animale, de production de lignées cellulaires ayant un haut rendement d'excrétion des particules selon l'invention et des résultats susceptibles d'être obtenus. On se reportera également aux dessins dans lesquels :
- les fig. 1 et 2 présentent les étapes successives essentielles de la production d'un plasmide conforme à l'invention, particulièrement appropriées à la transformation efficace de cellules animales, telles que la lignée CHO ;
- la fig. 3 est une représentation schématique des opérations d'amplification des séquences d'ADN codant pour le polypeptide immunogène selon l'invention dans des cellules CHO ;
- les fig. 4 et 5 font apparaître les résultats de l'amplification génique des cellules précédemment transformées dans les conditions du procédé selon l'invention.
- la fig. 6 contient une courbe représentative de la variation de la quantité d'antigène HBsAg produite en fonction des teneurs en ARN-HBV-spécifiques observables dans les cellules productrices ;
- la fig. 7 rapporte de façon schématique les résultats des récoltes successives effectuées sur des cellules en phase stationnaire.

### I - CONSTRUCTION DES VECTEURS

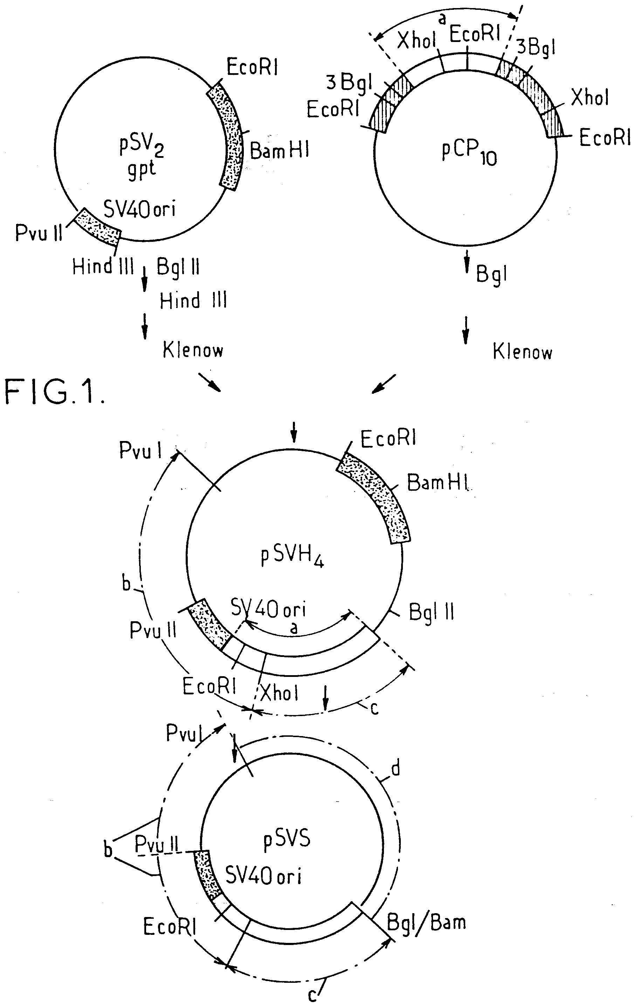

### A - Construction du plasmide pSVS (fig. 1)

Il permet l'expression de la région S (région pré-S + gène S) sous contrôle du promoteur précoce du virus SV40.

Le fragment BglII de 2,3 kb a été excisé du plasmide pcP10 décrit dans la demande de brevet européen n° 81 400634. Le plasmide pCP10 est schématisé dans la fig. 1. Les parties épaisses et hachurées sont originaires de DNA-HBV et la partie en trait plus mince provient du plasmide pBR322. La partie au regard de l'arc a correspond au susdit fragment de 2,3 kb. pCP10 contient un dimère en tandemn tête à queue du génome HBV. Ce fragment commence dix nucléotides avant l'ATG de la région pré-S et se termine 1,1 kb après le codon stop TAA du gène S. Il contient le site putatif de polyadénylation de l'ARNm de l'HBsAg, et le site d'initiation de transcription du gène S.

On utilise le promoteur précoce de SV40 contenu dans le plasmide pSV2 gpt (ATCC 37145) dans lequel le gène de la gpt de E. coli est fusionné à un fragment PvuII HindIII de 348 bp de la région précoce de SV40. Ce fragment comprend, outre l'origine de réplication de SV40 (SV40 ori), les promoteurs précoces et tardifs, le site d'initiation de transcription des messagers précoces et les 72 bp répétées en tandem. pSV2 gpt est schématisé dans la fig. 1. Les parties épaisses noires sont originaires de SV40, les parties en traits plus minces de pBR322. Les parties épaisses blanches contiennent les régions pré-S et S. Les parties hachurées correspondent aux séquences de l'HBV non utilisé.

Le fragment BglII de 2,3 kb, issu de pCP10, a été inséré dans le site unique HindIII du plasmide pSV2 gpt par ligature des extrémités des fragments d'ADN, rendues franches par l'ADN polymérase (Klenow). Après étude des plasmides recombinants un clone a été sélectionné (pSVH4) dans lequel la région codante pour l'HBS était orientée par rapport au promoteur SV40, de façon à permettre son expression. La construction a été vérifiée par cartes de restriction et la séquence nucléotidique à la jonction des deux fragments SV40 et HBV a été déterminée.

Le plasmide pSVS a été construit par ligature de trois fragments issus de trois plasmides :
1°) le fragment de 2,5 kb, PvuI-XhoI issu de pSVH4 (arc b),
2°) le fragment de 1,9 kb, XhoI BglII, issu de pcP10 (arc c),
3°) le fragment de 1,0 kb, bamHI PvuI, de pBR322 (arc d).
Le plasmide résultant, pSVS (5,4 kb) diffère de pSVH4 par l'absence de la région gpt et des séquences d'ADN SV40 adjacentes en aval de cette séquence gpt, et par la présence du fragment EcoRI-BamHI de pBR322.

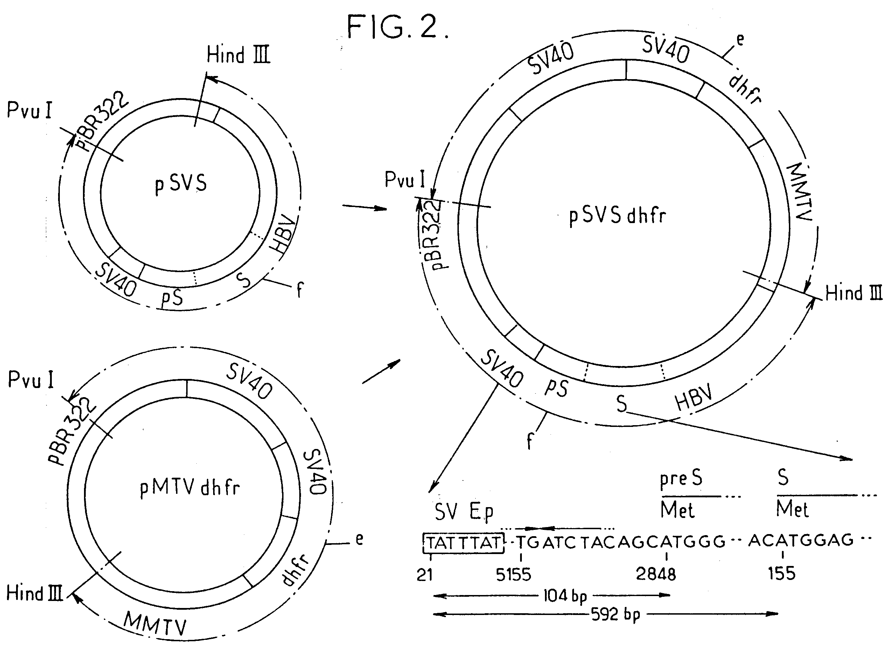

### B - Construction du plasmide pSVS dhfr (fig. 2)

On procède par recombinaison génétique des fragments définis sous a) et b) ci-après.

### a) Le fragment contenant la dhfr

Le plasmide pMTV dhfr décrit par G. Ringold et Coll. (1981) J. of Molecular and Applied Genetics 1 : 165-170 (déposé à la C.N.C.M. le 7.03.84 sous le n° I-286), après linéarisation par l'enzyme PvuI, a été ensuite digéré partiellement par l'enzyme HindIII, libérant plusieurs fragments dont un de 4400 bp (arc e) contenant :
- la LTR du MMTV,
- l'ADNc de la dhfr,
- l'intron du tAg de SV40,
- le site de polyadénylation des gènes précoces de SV40,
- un fragment EcoRI-PvuI du pBR322.

### b) Le fragment de DNA HBV

Le plasmide pSVS a été digéré par PvuI puis HindIII, libérant ainsi un fragment de 4676 bp (arc f) contenant :
- le fragment PvuI-PvuII contenant l'origine de réplication de pBR322 ;
- l'origine de réplication et le promoteur précoce du virus SV40 ;
- le fragment BglII 2,3 kb de l'HBV contenant les parties codant pour le pré-S et le gène S, ainsi que le signal de polyadénylation de ce gène ;
- le fragment BamHI-HindIII du pBR 322.

### c) Ligature des fragments

Après purification, ces deux fragments sont joints par les sites homologues PvuI et HindIII, rétablissant ainsi la résistance à l'ampicilline du pBR322.

Dans le vecteur recombinant, les unités de transcription de la région S et du gène dhfr sont orientées dans le même sens et séparées par environ 300 bp de pBR322.

Le cADN codant pour la dhfr peut aussi être obtenu à partir de pSV2 dhfr (ATCC 37146), pSV3 dhfr (ATCC 37147) ou pSV5 dhfr (ATCC 31148).

Le placement du gène de la dhfr sous le contrôle d'un promoteur faible (LTR du MMTV) et du gène HBV sous le contrôle d'un promoteur fort (promoteur précoce du SV40) augmente l'efficacité de l'amplification génique. La séquence nucléotidique de la jonction entre la région pré-S et le promoteur précoce du SV40 a été vérifiée (fig. 2).

### II - TRANSFECTION DES CELLULES ANIMALES

### 1°) Transfert et expression du plasmide pSVS dhfr dans des cellules CHO dhfr⁻ :

Des cellules CHO dhfr⁻ (Urlaub G. et Chasin L.A. (1980) P.N.A.S. 77, 4216-4220) (déposées à la C.N.C.M. le 7.03.84 et sous le n° I-287) ont été transfectées selon la technique de Graham et Van der Eb (1973, Virology 52, 456-467) modifiée par Parker et Stark (1979, J. Virology, 31, 360-369). La production d'HBsAg par les clones CHO dhfr⁺ a été testée par radioimmunoessai (RIA) dans le surnageant des cultures cellulaires. 60 % des clones dhfr⁺ étaient HBsAg⁺. Le taux de production d'HBsAg était relativement faible compris entre 1 à 20 ng/10⁶ cellules par 24 heures.

### 2°) Amplification des séquences HBV :

Elle a été obtenue en propageant les clones CHO HBsAg⁺ en présence de méthotrexate (MTX), analogue de l'acide folique et inhibiteur de la dhfr. En effet, la résistance au MTX est due principalement à une amplification du nombre de copies du gène dhfr, ce qui conduit à une augmentation de la quantité de l'enzyme dhfr. Les séquences HBV et le gène dhfr étant portés par le même plasmide et donc intégrées ensemble dans le DNA de la cellule-hôte, celles-ci sont co-amplifiées avec les séquences dhfr dans les clones résistant au MTX. Comme pour la dhfr, l'augmentation du nombre de copies de la séquence HBV s'accompagne d'une augmentation de synthèse d'HBsAg par la cellule.

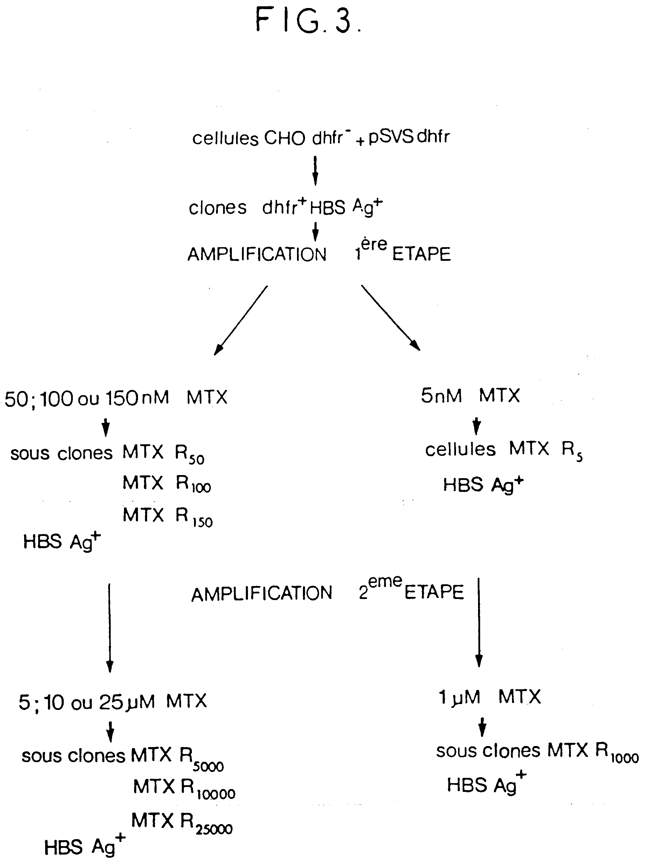

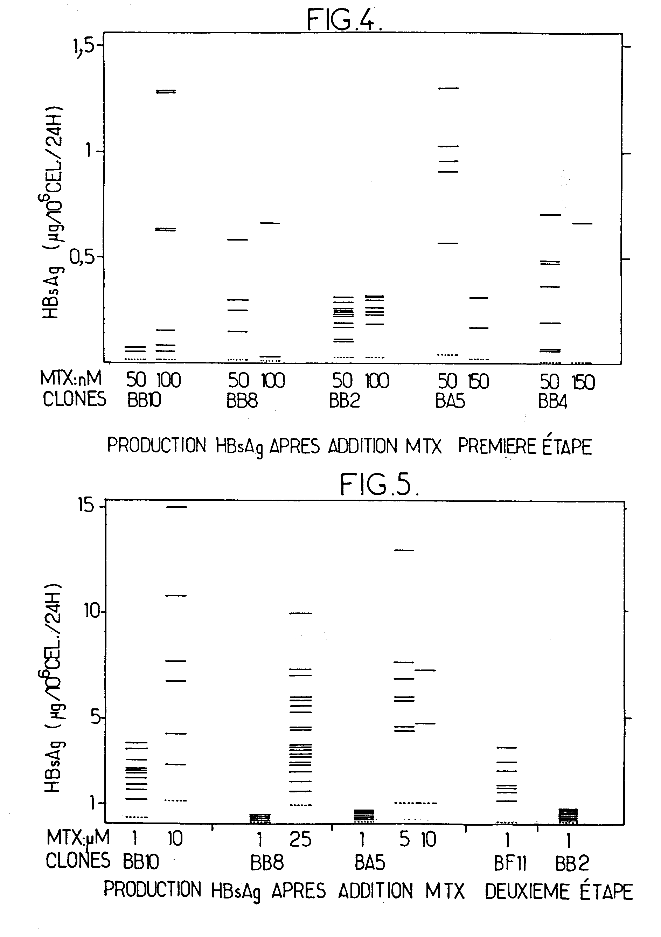

L'amplification génique a été effectuée en deux étapes selon la stratégie décrite sur la fig. 3. La fig. 4 représente les résultats de la première étape d'amplification et la fig. 5 les résultats de la deuxième étape. Celle-ci a été effectuée à partir des clones les plus producteurs, issus de la première étape. Dans les fig. 4 et 5, les barres en pointillés représentent le taux de synthèse d'HBsAg avant amplification et les barres en traits pleins après amplification. Les concentrations en MTX sont rapportées sur ces figures. Elles étaient de 50, 100 ou 140 nM dans la première étape et de 1,5, 10 ou 25 micro-M dans la deuxième. Au total, environ 150 clones résistant au MTX ont été criblés pour la production d'HBsAg. Comme le montrent les fig. 4 et 5, l'amplification de production d'HBsAg était très variable d'un clone à l'autre, aussi bien dans la première que dans la seconde étape.

Le nombre de copies par cellule de la séquence HBV présentes dans différents clones a été évalué selon la technique d'hybridation sur filtre de cellulose ou analogue, selon la technique dite du "dot blot", en utilisant comme sonde le DNA-HBV cloné marqué au ³²P. Pour une même dose de MTX, ce nombre était extrêmement variable d'un clone à l'autre, allant de 100 à 500.

L'organistion des séquences HBV dans les clones cellulaires a été analysée selon la technique de Southern. Les séquences HBV étaient présentes sous forme intégrée ; les profils électrophorétiques étaient assez comparables d'un clone à l'autre. Les différences observées entre des clones sélectionnés à une même dose de MTX ne permettaient pas d'expliquer la discordance entre le nombre de copies de DNA-HBV et le niveau de synthèse d'HBsAg (résultats non rapportés). Une analyse comparable a été effectuée pour le gène dhfr. Pour une même dose de MTX, le nombre de copies par cellule était également très variable d'un clone à l'autre (résultats non rapportés). Pour un clone donné, il y avait une amplification parallèle des séquences HBV et dhfr.

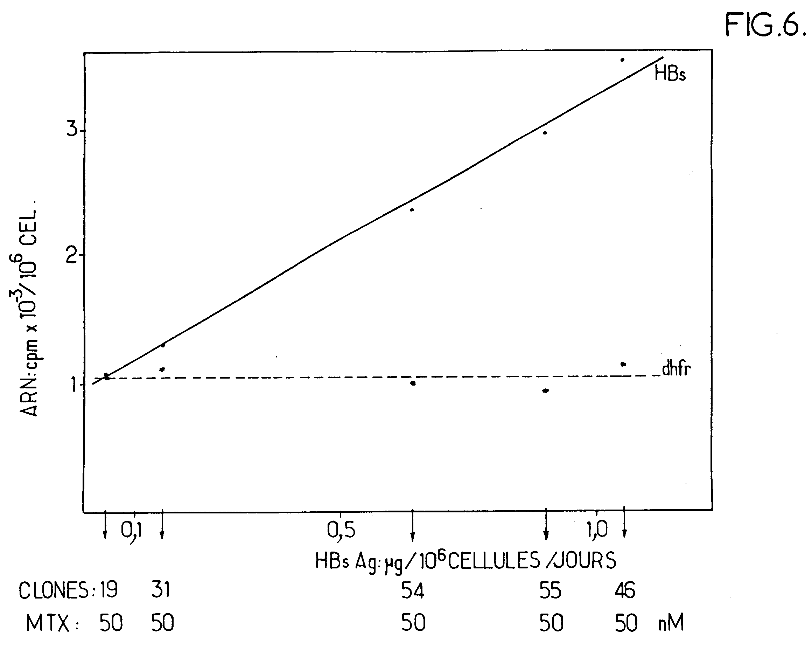

La quantité de RNA spécifiques de l'HBV a été également analysée par hybridation moléculaire. Contrairement aux résultats obtenus pour le DNA, la quantité de RNA-HBV-spécifiques était proportionnelle au taux de production d'HBsAg (fig. 6). D'autre part, pour une même dose de MTX (50 nano-M), la quantité de RNA dhfr-spécifiques était constante dans les différents clones analysés (fig. 6). Les RNA-HBV-spécifiques ont été analysés selon la technique du Northern. Deux RNA, l'un majeur de 2,1 kb, l'autre mineur de 2,5 kb, ont été mis en évidence. L'étude de ces RNA par cartographie à la nucléase S1 a montré l'existence d'une initiation majeure dans la région pré-S à la position 3157 correspondant au RNA 2,1 kb, l'autre mineure dans le promoteur du SV40 correspondant au RNA 2,5 kb.

### 3°) Analyse des particules HBsAg :

L'analyse a été effectuée sur les particules produites par le clone 37BA5. Des surnageants correspondant à plusieurs récoltes de 24 heures obtenues en phase stationnaire ont été rassemblés. Les particules HBsAg ont été purifiées par deux ultracentrifugations successives en gradient de densité de CsCl suivies d'une ultracentrifugation de vélocité en gradient de saccharose. Les particules HBsAg avaient une densité de 1,20. Observées au microscope électronique, elles apparaissaient comme des particules sphériques d'un diamètre moyen de 22 nm, morphologiquement semblables aux particules d'origine humaine. Aucune forme tubulaire n'a été observée.

Après dissociation des particules à 100° pendant 5 minutes en présence de DTT, les polypeptides ont été analysés par électrophorèse en gel de polyacrylamide en présence de SDS. Le gel a été révélé par coloration aux sels d'argent. Trois bandes ont été observées, correspondant à des polypeptides de 22.300, 26.100 et 34.000 daltons. L'intensité relative de la coloration de ces trois bandes a permis d'évaluer à 54 %, 19 % et 27 % les proportions de ces trois protéines. Après marquage in vivo par la ³⁵S méthionine, les particules purifiées ont été immunoprécipitées par un sérum anti-HBs, puis les protéines ont été analysées par électrophorèse. Les trois polypeptides décrits ci-dessus étaient détectés sur l'autoradiogramme et dans les mêmes proportions.

La présence du récepteur pour la pHSA à la surface des particules a été testée par radioimmunoessai en phase solide selon la technique de Hansson et Purcell ((1979), Infect. Immun. 26, 125), modifiée par Pontisso et al (J. of Virological Methods 6, 151-159). La détection de ce récepteur s'est révélée positive aussi bien dans le surnageant cellulaire que sur des particules purifiées (tableau 1) :

**TABLEAU I**

| | |
|---|---|
| Surnageant de culture (13 microgrammes/ml) | 2.805 cpm |
| Particules purifiées (12 microgrammes/ml) | 1.564 cpm |
| Témoin positif sérum HBe positif | 2.536 cpm |
| Témoin négatif sérum HBsAg négatif | 678 cpm |

On peut également utiliser la technique décrite par Machida et al (1983, Gastroenterology 85, 268-274.

### III - ESSAIS D'IMMUNISATION

Après addition d'hydroxyde d'aluminium Al(OH)₃ à la concentration de 0,1 %, une préparation de particules a été utilisée pour immuniser des souris Balb/c. Comme le montre le tableau II, le pouvoir immunogène des particules cellulaires est identique à celui du vaccin contre l'hépatite B, commercialisé sous la marque HEVAC B (et obtenu à partir de donneurs humains de sérums ayant des teneurs en HBsAg naturel). La dose efficace 50 % est de 0,04 microgramme pour les particules cellulaires et de 0,03 microgramme pour l'HEVAC B (marque Institut Pasteur).

**TABLEAU II**

| HBsAg Doses injectées | Vaccin HEVAC B | | Particules HBsAg CHO | |
|---|---|---|---|---|
| | Séroconversion > 50 uRIA Nombre de souris | Anticorps anti-HBs Moyenne géométrique uRIA | Séroconversion > 50 uRIA Nombre de souris | Anticorps anti-HBs Moyenne géométrique uRIA |
| 0,312 microgrammes | 20/20 | > 388 | 20/20 | > 505 |
| 0,078 microgrammes | 19/20 | > 212 | 14/20 | > 87 |
| 0,019 microgrammes | 6/20 | > 24 | 6/20 | > 24 |
| 0 témoins | 4/20 | | | |
| | DE₅₀0,04 microgrammes | | DE₅₀0,03 microgrammes | |
| uRIA = unités définies dans le test AUSAB commercialisé par ABBOTT | | | | |

Il résulte donc de ce qui précède que le vecteur pSVS dhfr permet, après intégration dans le DNA cellulaire, la synthèse et l'excrétion d'enveloppes vides du virus HBV sous forme de particules de 22 nm. L'élimination d'une grande partie du génome de l'HBV et en particulier du gène codant pour la protéine de la capside exclut la production de particules virales complètes infectieuses. Ce vecteur porte également une unité de transcription du gène de la dhfr murine qui, après introduction dans des cellules dhfr⁻, leur permet de se multiplier en présence de MTX par un phénomène d'amplification génique.

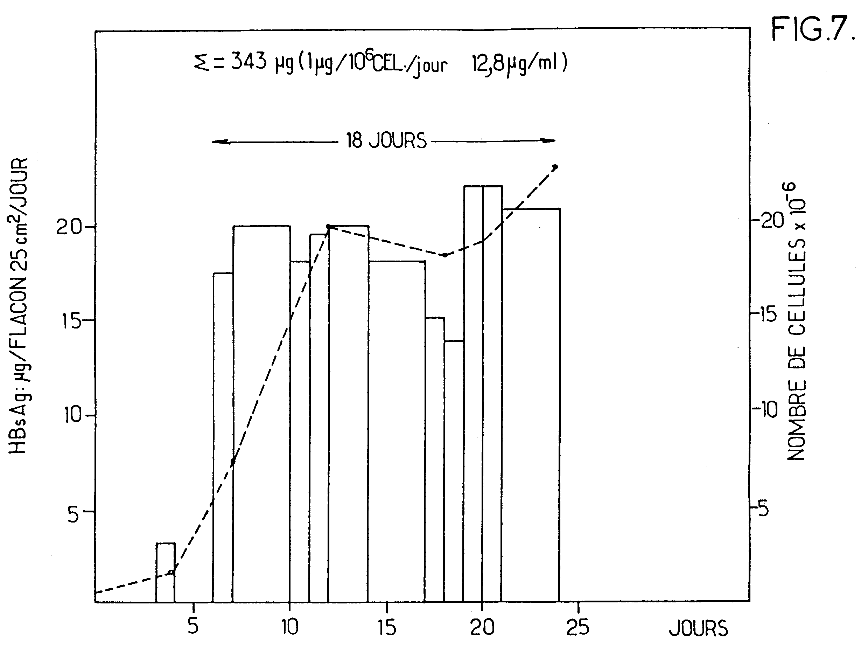

La technique d'amplification par la résistance au MTX utilisée ici a permis d'obtenir des clones de cellules CHO produisant des particules HBsAg à un niveau élevé. Cette amplification était variable d'un clone à l'autre. Dans certains cas, le taux de synthèse a été multiplié par 1500. Pour le clone concerné, le taux de production était de 15 microgrammes/10⁶ cellules par 24 heures. Plusieurs clones produisant de 1 à 10 microgrammes d'HBsAg/10⁶ cellules par 24 heures ont été obtenus. Le taux de production d'HBsAg exprimé par ml et par 24 heures dépend des conditions de culture. Des valeurs de 10 à 20 microgrammes/ml/24 heures ont pu être obtenues couramment, valeurs comparables à la concentration d'HBsAg dans le sérum des porteurs chroniques. Une étude cinétique de la production d'HBsAg a montré que cette synthèse reste constante en phase stationnaire pendant plusieurs semaines. La fig. 7 rapporte les résultats de récoltes successives effectuées sur des cellules en phase stationnaire, par renouvellement périodique du milieu de culture pendant 3 semaines. Du milieu sans sérum peut être utilisé pour ces récoltes. Outre l'impact sur le prix de revient, l'utilisation d'un milieu sans sérum facilite beaucoup la purification des particules à partir du surnageant cellulaire et élimine des contaminations éventuelles d'origine sérique. La synthèse d'HBsAg par les clones CHO s'est révélée très stable même en l'absence de MTX. Ceci sur une période de 6 mois, soit environ 300 générations. Dans certains cas, une légère augmentation spontanée de l'HBsAg produit a même été observée. Jamais une diminution ou une perte de la production d'HBsAg n'a été observée pendant l'amplification génique, ceci sur environ 150 clones testés. Ces deux dernières observations peuvent être dues à la structure du vecteur dans lequel les séquences HBV et le gène dhfr sont seulement séparés par environ 300 bp du pBR322.

L'invention permet par conséquent l'obtention de clones producteurs de particules immunogènes d'une stabilité génétique particulièrement remarquable.

Une corrélation entre le nombre de copies de séquences HBV et le taux de synthèse d'HBsAg n'a pas été observée. La technique utilisée (le "dot blot") permet une quantification globale, mais ne permet pas de discriminer les séquences fonctionnelles. Les résultats obtenus peuvent s'expliquer par l'apparition de délétions et de réarrangements dans le vecteur au cours de la transfection et de l'amplification. Cette hypothèse est supportée par la complexité des profils électrophorétiques des séquences HBV et dhfr intégrées. Beaucoup de ces séquences sont probablement non fonctionnelles. Par contre il y a parfaite proportionnalité entre le niveau de synthèse d'HBsAg et la quantité de RNA-HBV-spécifiques. Ceci démontre que l'augmentation de synthèse d'HBsAg observée est bien due à une augmentation de la transcription des séquences HBV, due en partie très vraisemblablement à une augmentation du nombre de copies fonctionnelles. Ce résultat montre par ailleurs que l'évaluation du nombre de copies d'un gène amplifié, que ce soit par dot-blot ou par analyse électrophorétique n'est pas le meilleur test pour évaluer le nombre de copies efficaces dans un clone cellulaire. Par contre, la quantification des RNA spécifiques reflète parfaitement les copies fonctionnelles. Cette quantification prend tout son intérêt dans les systèmes où la protéine produite n'est pas facilement dosable.

L'invention concerne donc plus particulièrement des clones contenant une dose d'ARN-HBV d'au moins 2 x 10³ cpm/10⁶ cellules dans le système de mesure tel qu'il a été décrit plus haut pour une sonde ayant une activité spécifique de 10⁸ cpm/microgramme (marquage au ³²P).

Deux RNA-HBV-spécifiques ont été mis en évidence. L'initiation du transcript majeur de 2,1 kb a été localisée dans la région pré-S à la position 3157. Ceci est en accord avec les résultats de Catteneo et al (1983) Nature, vol. 305, 336-338, qui ont localisé l'initiation de transcription du gène S à cette position. Le RNA mineur de 2,5 kb est initié dans le promoteur du SV40. Cet RNA pourrait être le mRNA du polypeptide de 34.000 (GP34) présent dans les particules. Il est donc vraisemblable que la synthèse du GP34 en quantité importante (27 % des polypeptides des particules) est due à l'utilisation du promoteur précoce du SV40 incluant les 72 bp répétées et placé en amont de la région pré-S.

L'utilisation d'un radioimmunoessai a montré que les particules synthétisées par les cellules CHO portent le récepteur de la pHSA. D'après Machida et al (1983) Gastroenterology 85, 268-274, ce récepteur serait porté par les polypeptides GP31 et GP35. Il est donc vraisemblable que dans notre système le récepteur est porté par le GP34. L'existence observée d'un seul polypeptide, et non de deux, porteur du récepteur de la pHSA dans les particules d'origine cellulaire n'est pas expliquée. Il est possible que, contrairement aux particules sériques, la glycosylation dans les cellules CHO soit homogène.

Les particules HBsAg de 22 nm synthétisées par les cellules CHO ont la même immunogénicité que l'HEVAC B qui est une préparation de particules d'origine sérique. Les taux de production d'HBsAg obtenus sont compatibles avec une utilisation industrielle. La présence du récepteur de la pHSA à la surface de ces particules est un argument supplémentaire à l'utilisation de ce système pour la fabrication d'un vaccin contre l'hépatite B.

A ce titre, l'invention concerne toute composition de vaccin contre l'hépatite virale B contenant une dose efficcace de particules conformes à l'invention, notamment de 3 à 6 microgrammes de protéine/ml, de préférence 5 microgrammes de protéine/ml (dose unitaire), en association avec un véhicule pharmaceutique approprié au mode d'administration choisi, notamment par voie parentérale.

## Revendications (Revendications pour l'(les) Etat(s) contractant(s) suivant(s): BE, CH, DE, GB, IT, LI, LU, NL, SE)

1. Composition utile pour la fabrication de vaccins, contenant ou formée par des particules polypeptidiques, sensiblement sphériques, au moins en ce qui concerne la plupart d'entre elles, ayant des propriétés immunogéniques et immunologiques caractéristiques de l'antigène HBsAg, ayant des tailles de 18 à 25 nm, notamment de 20 à 22 nm, et des densités permettant leur isolement dans une zone de densité à base de CsCl, et un niveau de pureté totale pour ce qui est de l'absence de toute particule de Dane et d'antigènes HBc, ladite composition étant caractérisée à la fois par l'absence d'antigènes HBe et par la présence de polypeptides ayant des poids moléculaires, notamment de l'ordre de 34.000 daltons, et portant les sites immunogènes aussi bien de l'antigène HBsAg que du récepteur de l'albumine humaine polymérisée du virus de l'hépatite B, dans une proportion supérieure à 10 % de la quantité totale des polypeptides constitutifs des susdites particules, ces particules étant susceptibles d'être obtenues parmi les produits d'excrétion de cellules CHO, transformées par un vecteur modifié par une séquence contenant les régions S et pré-S du génome du virus de l'hépatite B sous le contrôle d'un promoteur exogène efficace.

2. Composition selon la revendication 1, caractérisée par une proportion de polypeptides ayant des poids moléculaires de l'ordre de 34.000, supérieures à 20 % de la quantité totale des polypeptides constitutifs des susdites particules.

3. Composition selon la revendication 1 ou 2, caractérisée en ce qu'elle est exempte de tout composant sérique d'origine humaine.

4. Procédé de production d'une composition conforme à l'une quelconque des revendications 1 à 3, caractérisé :
- par la culture de cellules CHO transformées par un vecteur contenant une séquence d'ADN correspondant à la région S du génome du virus de l'hépatite virale B, caractérisé en ce que ladite séquence d'ADN est précédée par une séquence correspondant à la région pré-S du virus de l'hépatite virale B, l'ensemble des séquences correspondant à ces régions S et pré-S étant, à l'intérieur de ce vecteur, placé sous le contrôle direct d'un promoteur exogène possédant la capacité de permettre l'initiation efficace de la transcription des gènes directement sous son contrôle dans les susdites cellules de mammifère, ce promoteur étant en outre capable d'être incorporé avec lesdites séquences dans le génome desdites cellules CHO ou de leur conférer la capacité d'excréter des particules polypeptidiques ayant les propriétés immunogéniques de l'antigène HBsAg et du récepteur de la serum-albumine humaine polymérisée du virus de l'hépatite B, et
- par la récupération des particules polypeptidiques susdites.

5. Procédé selon la revendication 4, caractérisé en ce que le susdit promoteur est issu du virus SV40, ce promoteur correspondant notamment au promoteur précoce du virus SV40.

6. Procédé selon la revendication 4 ou la revendication 5, caractérisé en ce que la susdite séquence d'ADN contenant les susdites régions S et pré-S est placée, en phase, immédiatement derrière un fragment d'ADN contenant le promoteur et une séquence activatrice associée à ce premier promoteur.

7. Procédé selon la revendication 6, caractérisé en ce que le susdit fragment comprend de 300 à 400 paires de bases.

8. Procédé selon l'une quelconque des revendications 4 à 7, caractérisé en ce que le vecteur contient en outre une séquence d'ADN codant pour un marqueur, tel qu'une enzyme, lui-même sous le contrôle d'un promoteur distinct, ce second promoteur étant plus faible que le susdit premier promoteur.

9. Procédé selon la revendication 8, caractérisé en ce que le marqueur est constitué par un gène codant pour la dihydrofolate réductase.

10. Procédé selon la revendication 8 ou la revendication 9, caractérisé en ce que le second promoteur provient du virus de la tumeur mammaire de la souris (MMTV).

11. Lignées de cellules CHO susceptibles d'être maintenues en culture, productrice des particules polypeptidiques de la composition selon l'une quelconque des revendications 1 à 3, caractérisées en ce qu'elles sont transformées par un vecteur tel qu'il a été défini dans l'une quelconque des revendications 4 à 10.

12. Lignées formées de cellules conformes à la revendication 11, caractérisées par leur capacité à excréter dans leur milieu de culture des particules telles que définies dans l'une quelconque des revendications 1 à 3, à raison d'au moins 1 microgramme, et de préférence au moins 10 microgrammes d'antigène HBsAg par 10⁶ cellules et par 24 heures.

13. Lignées selon la revendication 11 ou la revendication 12, caractérisées par la présence que l'on peut y détecter d'ARN-HBV-spécifiques qui ont des tailles supérieures à 2,1 kb, notamment de l'ordre de 2,2 à 2,8 kb.

14. Lignées selon la revendication 13, caractérisées par la présence que l'on peut y détecter, d'ARN-HBV qui ont une taille de l'ordre de 2,4 à 2,5 kb.

15. Lignées selon la revendication 13 ou la revendication 14, caractérisées par la présence, détectable simultanément, d'un ARN-HBV spécifique ayant une taille de l'ordre de 2,1 kb, tel que celui qui résulte de la transcription des régions S et pré-S, normalement initié dans la région pré-S.

16. Procédé de production de lignées de cellules CHO conformes à l'une quelconque des revendications 12 à 15, caractérisé par la transformation de lignées cellulaires CHO susceptibles d'être maintenues en culture avec un vecteur tel qu'il a été défini dans l'une quelconque des revendications 4 à 10 et par l'isolement des cultures exprimant les régions S et pré-S du génome du virus de l'hépatite B.

17. Procédé selon la revendication 16, caractérisé en ce que le vecteur est tel que défini dans l'une quelconque des revendications 8 à 10, et en ce que la culture des lignées cellulaires transformées est réalisée en présence d'un inhibiteur du marqueur dont la teneur dans le milieu de culture est réglée à une concentration suffisante pour provoquer une amplification du nombre de copies du gène codant pour le marqueur dans certaines au moins des colonies mises en culture, cette amplification du nombre de gènes codant pour le marqueur permettant, à la fois, la sélection de colonies résistantes et l'obtention de clones cellulaires sécréteurs de quantités accrues des susdites particules.

18. Procédé selon la revendication 17, caractérisé en ce que le marqueur est constitué par une séquence ou un gène codant pour la dihydrofolate-réductase et en ce que l'inhibiteur est constitué par le méthotrexate.

19. Vaccin contenant une dose efficace de la composition selon l'une quelconque des revendications 1 à 3, en association avec un véhicule pharmaceutique approprié au mode d'administration choisi.

## Revendications (Revendications pour l'(les) Etat(s) contractant(s) suivant(s): AT)

1. Procédé pour la production d'une composition utile pour la fabrication de vaccins, contenant ou formée par des particules polypeptidiques, produites par une technique de génie génétique, sensiblement sphériques, au moins en ce qui concerne la plupart d'entre elles, ayant des propriétés immunogéniques et immunologiques caractéristiques de l'antigène HBsAg, ayant des tailles de 18 à 25 nm, notamment de 20 à 22 nm, et des densités permettant leur isolement dans une zone de densité à base de CsCl, et un niveau de pureté totale pour ce qui est de l'absence de toute particule de Dane et d'antigènes HBc, ladite composition contenant des polypeptides ayant des poids moléculaires, notamment de l'ordre de 34.000 daltons, et portant les sites immunogènes aussi bien de l'antigène HBsAg que du récepteur de l'albumine humaine polymérisée, dans une proportion supérieure à 10 % de la quantité totale des polypeptides constitutifs des susdites particules, ce procédé étant caractérisé :
- par la culture de cellules CHO transformées par un vecteur contenant une séquence d'ADN correspondant à la région S du génome du virus de l'hépatite virale B, caractérisé en ce que ladite séquence d'ADN est précédée par une séquence correspondant à la région pré-S du virus de l'hépatite virale B, l'ensemble des séquences correspondant à ces régions S et pré-S étant, à l'intérieur de ce vecteur, placé sous le contrôle direct d'un promoteur exogène possédant la capacité de permettre l'initiation efficace de la transcription des gènes directement sous son contrôle dans les susdites cellules CHO, ce promoteur étant en outre capable de leur conférer la capacité d'excréter des polypeptides ayant les propriétés immunogéniques de l'antigène HBsAg et du récepteur de la serum-albumine humaine polymérisée, et
- par la récupération des particules polypeptidiques susdites.

2. Procédé selon la revendication 1, caractérisé en ce que le susdit promoteur est issu du virus SV40, ce promoteur correspondant notamment au promoteur précoce du virus SV40.

3. Procédé selon la revendication 1 ou la revendication 2, caractérisé en ce que la susdite séquence d'ADN contenant les susdites régions S et pré-S est placée, en phase, immédiatement derrière un fragment d'ADN contenant le promoteur et une séquence activatrice associée à ce premier promoteur.

4. Procédé selon la revendication 3, caractérisé en ce que le susdit fragment comprend de 300 à 400 paires de bases.

5. Procédé selon l'une quelconque des revendications 1 à 4, caractérisé en ce que le vecteur contient en outre une séquence d'ADN codant pour un marqueur, tel qu'une enzyme, lui-même sous le contrôle d'un promoteur distinct, ce second promoteur étant plus faible que le susdit premier promoteur.

6. Procédé selon la revendication 5, caractérisé en ce que le marqueur est constitué par un gène codant pour la dihydrofolate réductase.

7. Procédé selon la revendication 6, caractérisé en ce que le second promoteur provient du virus de la tumeur mammaire de la souris (MMTV).

8. Procédé de production de lignées de cellules CHO susceptibles d'être maintenues en culture, et de produire des particules polypeptidiques de la composition telle que définie dans la revendication 1, caractérisée en ce qu'elles sont transformées par un vecteur tel qu'il a été défini dans l'une quelconque des revendications 1 à 7.

9. Procédé selon la revendication 7 ou 8, caractérisé en ce qu'après la susdite transformation, on sélectionne les colonies de cellules ayant la capacité à excréter dans leur milieu de culture des particules, à raison d'au moins 1 microgramme, et de préférence au moins 10 microgrammes d'antigène HBsAg par 10⁶ cellules et par 24 heures.

10. Procédé selon la revendication 8 ou la revendication 9, caractérisé en ce que le vecteur est tel que défini dans l'une quelconque des revendications 1 à 7, et en ce que la culture des lignées cellulaires transformées est réalisée en présence d'un inhibiteur du marqueur dont la teneur dans le milieu de culture est réglée à une concentration suffisante pour provoquer une amplification du nombre de copies du gène codant pour le marqueur dans certaines au moins des colonies mises en culture, cette amplification du nombre de gènes codant pour le marqueur permettant, à la fois, la sélection de colonies résistantes et l'obtention de clones cellulaires sécréteurs de quantités accrues des susdites particules.

11. Procédé selon la revendication 10, caractérisé en ce que le marqueur est constitué par une séquence ou un gène codant pour la dihydrofolate-réductase et en ce que l'inhibiteur est constitué par le méthotrexate.

12. Procédé de fabrication d'un vaccin contenant une dose efficace de la composition produite par le procédé selon l'une quelconque des revendications 1 à 7, caractérisé par l'association de cette composition avec un véhicule pharmaceutique approprié au mode d'administration choisi.

## Claims (Claims for the following Contracting State(s): BE, CH, DE, GB, IT, LI, LU, NL, SE)

1. Composition useful for the manufacture of vaccines, containing or formed of polypeptide particles which are substantially spherical, at least as regards most of them, having immunogenic and immunological properties characteristic of the HBsAg antigen, having sizes of 18 to 25 nm, especially of 20 to 22 nm, and densities which allow their isolation in a CsCl-based density zone, and a total purity level as regards the absence of any Dane particle and HBc antigens, the said composition being characterized both by the absence of HBe antigens and by the presence of polypeptides having molecular weights especially of the order of 34,000 daltons and carrying the immunogenic sites both of the HBsAg antigen and of the polymerized human albumin receptor of the hepatitis B virus, in a proportion greater than 10% of the total quantity of the constituent polypeptides of the abovementioned particles, these particles being capable of being obtained among the excretion products of CHO cells transformed by a vector modified by a sequence containing the S and pre-S regions of the genome of the hepatitis B virus under the control of an efficient exogenous promoter.

2. Composition according to Claim 1, characterized by a proportion of peptides having molecular weights of the order of 34,000 greater than 20% of the total quantity of the constituent polypeptides of the above-mentioned particles.

3. Composition according to Claim 1 or 2, characterized in that it is free of any serum component of human origin.

4. Process for the production of a composition in accordance with any one of Claims 1 to 3, characterized:
- by the culture of CHO cells transformed by a vector containing a DNA sequence corresponding to the S region of the genome of the viral hepatitis B virus, characterized in that the said DNA sequence is preceded by a sequence corresponding to the pre-S region of the viral hepatitis B virus, the entire sequences corresponding to these S and pre-S regions being, within this vector, placed under the direct control of an exogenous promoter having the capacity to allow the efficient initiation of transcription of the genes directly under its control in the abovementioned mammalian cells, this promoter being, in addition, capable of being incorporated with the said sequences into the genome of the said CHO cells or of conferring on them the capacity to excrete polypeptide particles having the immunogenic properties of the HBsAg antigen and of the polymerized human serum albumin receptor of the hepatitis B virus, and
- by the recovery of the abovementioned polypeptide particles.

5. Process according to Claim 4, characterized in that the abovementioned promoter is derived from the SV40 virus, this promoter corresponding especially to the SV40 virus early promoter.

6. Process according to Claim 4 or Claim 5, characterized in that the abovementioned DNA sequence containing the abovementioned S and pre-S regions is placed, in phase, immediately behind a DNA fragment containing the promoter and an activator sequence associated with this first promoter.

7. Process according to Claim 6, characterized in that the abovementioned fragment comprises from 300 to 400 base pairs.

8. Process according to any one of Claims 4 to 7, characterized in that the vector contains, in addition, a DNA sequence encoding a marker, such as an enzyme, itself under the control of a separate promoter, this second promoter being weaker than the abovementioned first promoter.

9. Process according to Claim 8, characterized in that the marker consists of a gene encoding the dihydrofolate reductase.

10. Process according to Claim 8 or Claim 9, characterized in that the second promoter is obtained from the mouse mammary tumour virus (MMTV).

11. CHO cell lines capable of being maintained in culture, which produce the polypeptide particles of the composition according to any one of Claims 1 to 3, characterized in that they are transformed by a vector as defined in any one of Claims 4 to 10.

12. Lines formed of cells in accordance with Claim 11, characterized by their capacity to excrete into their culture medium particles as defined in any one of Claims 1 to 3, in an amount of at least 1 microgram, and preferably at least 10 micrograms of HBsAg antigen per 10⁶ cells and per 24 hours.

13. Lines according to Claim 11 or Claim 12, characterized by the presence, which can be detected therein, of HBV-specific RNAs which have sizes greater than 2.1 kb, especially of the order of 2.2 to 2.8 kb.

14. Lines according to Claim 13, characterised by the presence, which can be detected therein, of HBV RNAs which have a size of the order of 2.4 to 2.5 kb.

15. Lines according to Claim 13 or Claim 14, characterized by the presence, detectable simultaneously, of a HBV-specific RNA having a size of the order of 2.1 kb, such as that which results from the transcription of the S and pre-S regions, normally initiated in the pre-S region.

16. Process for the production of CHO cell lines in accordance with any one of Claims 12 to 15, characterized by the transformation of CHO cell lines capable of being maintained in culture with a vector as defined in any one of Claims 4 to 10 and by the isolation of the cultures expressing the S and pre-S regions of the genome of the hepatitis B virus.

17. Process according to Claim 16, characterized in that the vector is as defined in any one of Claims 8 to 10, and in that the culture of the transformed cell lines is performed in the presence of an inhibitor of the marker whose content in the culture medium is adjusted to a concentration sufficient to cause amplification of the copy number of the gene encoding the marker in at least some of the colonies cultured, this amplification of the number of genes encoding the marker allowing both the selection of resistant colonies and the production of cell clones secreting increased quantities of the above-mentioned particles.

18. Process according to Claim 17, characterized in that the marker consists of a sequence or a gene encoding dihydrofolate reductase and in that the inhibitor consists of methotrexate.

19. Vaccine containing an effective dose of the composition according to any one of Claims 1 to 3, in combination with a pharmaceutical vehicle appropriate to the mode of administration chosen.

## Claims (Claims for the following Contracting State(s): AT)

1. Process for the production of a composition useful for the manufacture of vaccines, containing or formed of polypeptide particles produced by a genetic engineering technique, which are substantially spherical, at least as regards most of them, having immunogenic and immunological properties characteristic of the HBsAg antigen, having sizes of 18 to 25 nm, especially of 20 to 22 nm, and densities which allow their isolation in a CsCl-based density zone, and a total purity level as regards the absence of any Dane particle and HBc antigens, the said composition containing polypeptides having molecular weights especially of the order of 34,000 daltons and carrying the immunogenic sites both of the HBsAg antigen and of the polymerized human albumin receptor, in a proportion greater than 10% of the total quantity of the constituent polypeptides of the above-mentioned particles, this process being characterized:
- by the culture of CHO cells transformed by a vector containing a DNA sequence corresponding to the S region of the genome of the viral hepatitis B virus, characterized in that the said DNA sequence is preceded by a sequence corresponding to the pre-S region of the viral hepatitis B virus, the entire sequences corresponding to these S and pre-S regions being, within this vector, placed under the direct control of an exogenous promoter having the capacity to allow the efficient initiation of transcription of the genes directly under its control in the abovementioned CHO cells, this promoter being, in addition, capable of conferring on them the capacity to excrete polypeptide particles having the immunogenic properties of the HBsAg antigen and of the polymerized human serum albumin receptor, and
- by the recovery of the abovementioned polypeptide particles.

2. Process according to Claim 1, characterized in that the abovementioned promoter is derived from the SV40 virus, this promoter corresponding especially to the SV40 virus early promoter.

3. Process according to Claim 1 or Claim 2, characterized in that the abovementioned DNA sequence containing the abovementioned S and pre-S regions is placed, in phase, immediately behind a DNA fragment containing the promoter and an activator sequence associated with this first promoter.

4. Process according to Claim 3, characterized in that the abovementioned fragment comprises from 300 to 400 base pairs.

5. Process according to any one of Claims 1 to 4, characterized in that the vector contains, in addition, a DNA sequence encoding a marker, such as an enzyme, itself under the control of a separate promoter, this second promoter being weaker than the abovementioned first promoter.

6. Process according to Claim 5, characterized in that the marker consists of a gene encoding the dihydrofolate reductase.

7. Process according to Claim 6, characterized in that the second promoter is obtained from the mouse mammary tumour virus (MMTV).

8. Process for the production of CHO cell lines capable of being maintained in culture and of producing polypeptide particles of the composition as defined in Claim 1, characterized in that they are transformed by a vector as defined in any one of Claims 1 to 7.

9. Process according to Claim 7 or 8, characterized in that, after the abovementioned transformation, the colonies of cells are selected which have the capacity to excrete into their culture medium particles in an amount of at least 1 microgram, and preferably at least 10 micrograms of HBsAg antigen per 10⁶ cells and per 24 hours.

10. Process according to Claim 8 or Claim 9, characterized in that the vector is as defined in any one of Claims 1 to 7, and in that the culture of the transformed cell lines is performed in the presence of an inhibitor of the marker whose content in the culture medium is adjusted to a concentration sufficient to cause amplification of the copy number of the gene encoding the marker in at least some of the colonies cultured, this amplification of the number of genes encoding the marker allowing both the selection of resistant colonies and the production of cell clones secreting increased quantities of the abovementioned particles.

11. Process according to Claim 10, characterized in that the marker consists of a sequence or a gene encoding dihydrofolate reductase and in that the inhibitor consists of methotrexate.

12. Process for the manufacture of a vaccine containing an effective dose of the composition produced by the process according to any one of Claims 1 to 7, characterized by the combination of this composition with a pharmaceutical vehicle appropriate to the mode of administration chosen.

## Patentansprüche (Patentansprüche für folgende(n) Vertragsstaat(en): BE, CH, DE, GB, IT, LI, LU, NL, SE)

1. Zusammensetzung, die nützlich ist für die Anfertigung von Vakzinen, enthaltend oder gebildet durch Polypeptidpartikel, die etwa sphärisch sind, zumindest was den Großteil von ihnen betrifft, mit für das HBsAg-Antigen charakteristischen immunogenen und immunologischen Eigenschaften, Größen von 18-25 nm, insbesondere 20 bis 22 nm, und Dichten, die ihre Isolierung in einer auf CsCl basierenden Dichtezone erlauben, sowie einem insgesamten Reinheitsgrad, was die Abwesenheit aller Dane-Partikel und HBc-Antigene betrifft, wobei die Zusammensetzung gleichzeitig gekennzeichnet ist durch die Abwesenheit von HBe-Antigenen und durch die Anwesenheit von Polypeptiden, die Molekulargewichte insbesondere im Bereich von 34 000 Dalton und die immunogenen Bereiche sowohl des HBsAg-Antigens als auch des Rezeptors von polymerisiertem menschlichen Albumin des Hepatitis-B-Virus aufweisen, in einem Verhältnis von mehr als 10 % der Gesamtmenge der Polypeptide, die Bestandteile der Partikel sind, wobei diese Partikel erhalten werden können von Ausscheidungsprodukten von CHO-Zellen, die mit einem Vektor transformiert sind, der modifiziert ist durch eine Sequenz, die die S- und prä-S-Regionen des Genoms des Hepatitis-B-Virus unter der Kontrolle eines wirksamen exogenen Promotors enthält.

2. Zusammensetzung nach Anspruch 1, gekennzeichnet durch einen Anteil von Polypeptiden mit Molekulargewichten im Bereich von 34 000 von mehr als 20 % der Gesamtmenge der Polypeptide, die Bestandteile der Partikel sind.

3. Zusammensetzung nach Anspruch 1 oder 2, dadurch gekennzeichnet, daß sie frei ist von allen Serumbestandteilen menschlichen Ursprungs.

4. Verfahren zur Herstellung einer Zusammensetzung gemäß einem der Ansprüche 1 bis 3, gekennzeichnet:
- durch die Kultivierung von CHO-Zellen, die mit einem Vektor transformiert sind, der eine der Region S des Genoms des viralen Heptatitis-B-Virus entsprechende DNA-Sequenz enthält und dadurch gekennzeichnet ist, daß dieser DNA-Sequenz eine Sequenz vorangeht, die der prä-S-Region des viralen Heptatitis-B-Virus entspricht, und daß die Anordnung der diesen Regionen S und prä-S entsprechenden Sequenzen, innerhalb des Vektors, unter die direkte Kontrolle eines exogenen Promotors gestellt ist, der die Fähigkeit besitzt, die wirksame Initiation der Transkription von direkt unter seiner Kontrolle stehenden Genen in den Säugerzellen zu erlauben, wobei dieser Promotor ferner mit den Sequenzen in das Genom der CHO-Zellen inkorporiert werden kann oder ihnen die Fähigkeit zur Ausscheidung von Polypeptidpartikeln mit den immunogenen Eigenschaften des HBsAg-Antigens und des Rezeptors des polymerisierten menschlichen Serumalbumins von Hepatitis-B-Virus verleihen kann, und
- durch die Gewinnung der Polypeptidpartikel.

5. Verfahren nach Anspruch 4, dadurch gekennzeichnet, daß der Promotor von dem SV40-Virus abstammt, und dieser Promotor insbesondere dem frühen Promotor des SV40-Virus entspricht.

6. Verfahren nach Anspruch 4 oder Anspruch 5, dadurch gekennzeichnet, daß die die S- und prä-S-Regionen enthaltende DNA-Sequenz unmittelbar hinter einem DNA-Fragment, in Phase, angeordnet ist, das den Promotor und eine mit diesem ersten Promotor assoziierte Aktivierungssequenz enthält.

7. Verfahren nach Anspruch 6, dadurch gekennzeichnet, daß das Fragment 300 bis 400 Basenpaare umfaßt.

8. Verfahren nach einem der Ansprüche 4 bis 7, dadurch gekennzeichnet, daß der Vektor ferner eine einen Marker, wie z.B. ein Enzym, codierende DNA-Sequenz enthält, ihrerseits unter der Kontrolle eines anderen Promotors, wobei dieser zweite Promotor schwächer ist als der erste Promotor.

9. Verfahren nach Anspruch 8, dadurch gekennzeichnet, daß der Marker aus einem Gen besteht, das die Dihydrofolatreduktase codiert.

10. Verfahren nach Anspruch 8 oder Anspruch 9, dadurch gekennzeichnet, daß der zweite Promotor vom Mause Mammary Tumor Virus (MMTV) abstammt.

11. CHO-Zellinien, die in Kultur aufrechterhalten werden können und die Erzeuger sind von Polypeptidpartikeln der wie in einem der Ansprüche 1 bis 3 definierten Zusammensetzung, dadurch gekennzeichnet, daß sie mit einem wie in einem der Ansprüche 4 bis 10 definierten Vektor transformiert sind.

12. Linien, die gebildet werden von Zellen gemäß Anspruch 11, gekennzeichnet durch ihre Fähigkeit in ihr Kulturmedium wie in einem der Ansprüche 1 bis 3 definierte Partikel auszuscheiden, nämlich mindestens 1 Mikrogramm, vorzugsweise mindestens 10 Mikrogramm HBsAG-Antigen pro 10⁶ Zellen und pro 24 Stunden.

13. Linien nach Anspruch 11 oder Anspruch 12, gekennzeichnet durch das nachweisbare Auftreten von HBV-spezifischen RNAs, die eine Größe von mehr als 2,1 kb haben, insbesondere im Bereich von 2,2 bis 2,8 kb.

14. Linien nach Anspruch 13, gekennzeichnet durch das nachweisbare Auftreten von HBV-RNAs, die eine Größe im Bereich von 2,4 bis 2,5 kb haben.

15. Linien nach Anspruch 13 oder Anspruch 14, gekennzeichnet durch die gleichzeitig nachweisbare Anwesenheit einer HBV-spezifischen RNA mit einer Größe im Bereich von 2,1 kb, wie z.B. diejenige, die aus der Transkription der Regionen S und prä-S resultiert, die normalerweise in der Region prä-S startet.

16. Verfahren zur Herstellung von CHO-Zellinien gemäß einem der Ansprüche 12 bis 15, gekennzeichnet durch die Transformation von CHO-Zellinien, die in Kultur aufrechterhalten werden können, mit einem Vektor wie er in einem der Ansprüche 4 bis 10 definiert wurde, und durch die Isolierung von Kulturen, die die Regionen S und prä-S des Hepatitis-B-Virusgenoms exprimieren.

17. Verfahren nach Anspruch 16, dadurch gekennzeichnet, daß der Vektor ein wie in einem der Ansprüche 8 bis 10 definierter Vektor ist und dadurch, daß die Kultivierung transformierter Zellinien in Gegenwart eines Inhibitors des Markers durchgeführt wird, dessen Gehalt im Kulturmedium in einer Konzentration eingestellt wird, die ausreicht, die Amplifizierung der Anzahl der Kopien des den Marker codierenden Gens hervorzurufen in wenigstens einigen der in Kultur genommenen Kolonien, wobei diese Amplifikation der Anzahl der den Marker codierenden Gene gleichzeitig die Selektion resistenter Kolonien erlaubt und die Gewinnung von Zellclonen, die gesteigerte Mengen der Partikel sekretieren.

18. Verfahren nach Anspruch 17, dadurch gekennzeichnet, daß der Marker aus einer Sequenz oder einem Gen besteht, die (das) die Dihydrofolatreduktase codiert, und dadurch, daß der Inhibitor aus Methotrexat besteht.

19. Vakzine, enthaltend eine wirksame Menge der Zusammensetzung nach einem der Ansprüche 1 bis 3 in Kombination mit einem für die gewählte Verabreichung angemessenem pharmazeutischen Vehikel.

## Patentansprüche (Patentansprüche für folgende(n) Vertragsstaat(en): AT)

1. Verfahren zur Herstellung einer für die Anfertigung von Vakzinen nützlichen Zusammensetzung, enthaltend oder gebildet durch gentechnisch hergestellte Polypeptidpartikel, die etwa sphärisch sind, zumindest was den Großteil von ihnen betrifft, mit für das HBsAg-Antigen charakteristischen immunogenen und immunologischen Eigenschaften, Größen von 18-25 nm, insbesondere 20 bis 22 nm, und Dichten, die ihre Isolierung in einer auf CsCl basierenden Dichtezone erlauben, sowie einem insgesamten Reinheitsgrad, was die Abwesenheit aller Dane-Partikel und HBc-Antigene betrifft, wobei die Zusammensetzung Polypeptide enthält, die Molekulargewichte insbesondere im Bereich von 34 000 Dalton und die immunogenen Bereiche sowohl des HBsAg-Antigens als auch des Rezeptors von polymerisiertem menschlichen Albumin aufweisen, in einem Verhältnis von mehr als 10 % der Gesamtmenge der Polypeptide, die Bestandteile der obengenannten Partikel sind, wobei dieses Verfahren gekennzeichnet ist:
- durch die Kultivierung von CHO-Zellen, die mit einem Vektor transformiert sind, der eine der Region S des Genome des viralen Heptatitis-B-Virus entsprechende DNA-Sequenz enthält und dadurch gekennzeichnet ist, daß dieser DNA-Sequenz eine Sequenz vorangeht, die der prä-S-Region des viralen Heptatitis-B-Virus entspricht, und daß die Anordnung der diesen Regionen S und prä-S entsprechenden Sequenzen innerhalb des Vektors unter die direkte Kontrolle eines exogenen Promotors gestellt ist, der die Fähigkeit besitzt, die wirksame Initiation der Transkription von direkt unter seiner Kontrolle stehenden Genen in den CHO-Zellen zu erlauben, wobei dieser Promotor ferner fähig ist, ihnen die Fähigkeit zur Ausscheidung der Polypeptide zu verleihen, die die immunogenen Eigenschaften des HBsAg-Antigens und des Rezeptors des polymerisierten menschlichen Serumalbumins haben, und
- durch die Gewinnung der Polypeptidpartikel.

2. Verfahren nach Anspruch 1, dadurch gekennzeichnet, daß der Promotor von dem SV40-Virus abstammt, und dieser Promotor insbesondere dem frühen Promotor des SV40-Virus entspricht.

3. Verfahren nach Anspruch 1 oder Anspruch 2, dadurch gekennzeichnet, daß die die S- und prä-S-Regionen enthaltende DNA-Sequenz unmittelbar hinter einem DNA-Fragment, in Phase, angeordnet ist, das den Promotor und eine mit diesem ersten Promotor assoziierte Aktivierungssequenz enthält.

4. Verfahren nach Anspruch 3, dadurch gekennzeichnet, daß das Fragment 300 bis 400 Basenpaare umfaßt.

5. Verfahren nach einem der Ansprüche 1 bis 4, dadurch gekennzeichnet, daß der Vektor ferner eine einen Marker, wie z.B. ein Enzym, codierende DNA-Sequenz enthält, ihrerseits unter der Kontrolle eines anderen Promotors, wobei dieser zweite Promotor schwächer ist als der erste Promotor.

6. Verfahren nach Anspruch 5, dadurch gekennzeichnet, daß der Marker aus einem Gen besteht, das die Dihydrofolatreduktase codiert.

7. Verfahren nach Anspruch 6, dadurch gekennzeichnet, daß der zweite Promotor vom Mause Mammary Tumor Virus (MMTV) abstammt.

8. Verfahren zur Herstellung von CHO-Zellinien, die in Kultur aufrechterhalten werden können und die Polypeptidpartikel der wie in Anspruch 1 definierten Zusammensetzung produzieren können, dadurch gekennzeichnet, daß sie mit einem wie in einem der Ansprüche 1 bis 7 definierten Vektor transformiert sind.

9. Verfahren nach Anspruch 7 oder 8, dadurch gekennzeichnet, daß nach der Transformation die Zellkolonien selektioniert werden, die in der Lage sind, in ihr Kulturmedium Partikel auszuscheiden, nämlich mindestens 1 Mikrogramm, vorzugsweise mindestens 10 Mikrogramm HBsAG-Antigen pro 10⁶ Zellen und pro 24 Stunden.

10. Verfahren nach Anspruch 8 oder Anspruch 9, dadurch gekennzeichnet, daß der Vektor ein wie in einem der Ansprüche 1 bis 7 definierter Vektor ist und dadurch, daß die Kultivierung transformierter Zellinien in Gegenwart eines Inhibitors des Markers durchgeführt wird, dessen Gehalt im Kulturmedium in einer Konzentration eingestellt wird, die ausreicht, die Amplifizierung der Anzahl der Kopien des den Marker codierenden Gens hervorzurufen in wenigstens einigen der in Kultur genommenen Kolonien, wobei diese Amplifikation der Anzahl der den Marker codierenden Gene gleichzeitig die Selektion resistenter Kolonien erlaubt und die Gewinnung von Zellclonen, die gesteigerte Mengen der Partikel sekretieren.

11. Verfahren nach Anspruch 10, dadurch gekennzeichnet, daß der Marker aus einer Sequenz oder einem Gen besteht, die (das) die Dihydrofolatreduktase codiert, und dadurch, daß der Inhibitor aus Methotrexat besteht.

12. Verfahren zur Anfertigung einer Vakzine, die eine wirksame Menge der gemäß dem Verfahren nach einem der Ansprüche 1 bis 7 hergestellten Zusammensetzung enthält, gekennzeichnet durch die Kombination dieser Zusammensetzung mit einem für die gewählte Verabreichung angemessenen pharmazeutischen Vehikel.
